# EUROPEAN PATENT SPECIFICATION

(11) **EP 1 273 299 B1**
(45) Date of publication and mention of the grant of the patent: **19.10.2005**
(21) Application number: 01919789.6
(22) Date of filing: 05.04.2001
(51) Int. Cl.: A61K 31/506, A61K 9/08, A61K 47/10, A61K 47/12, A61K 47/20, A61K 47/22, A61K 47/46, A61K 7/06, A61P 17/14

(54) **MINOXIDIL-CONTAINING PREPARATIONS**
MINOXIDIL ENTHALTENDE ZUBEREITUNG
PREPARATIONS A BASE DE MINOXIDIL

(30) Priority: 07.04.2000 JP 2000107028
(43) Date of publication of application: 08.01.2003
(73) Proprietor: Taisho Pharmaceutical Co. Ltd., Tokyo 170-8633 (JP)
(72) Inventor: IMAMURA, Koji, c/o TAISHO PHARMACEUTICAL CO. LTD., Toshima-ku, Tokyo 171-8633 (JP); OCHIAI, Rumi, c/o TAISHO PHARMACEUTICAL CO. LTD., Toshima-ku, Tokyo 171-8633 (JP); OKAJIMA, Takako, c/o TAISHO PHARMACEUTICAL CO. LTD, Toshima-ku, Tokyo 171-8633 (JP); MORIOKA, Susumu, c/o TAISHO PHARMACEUTICAL CO. LTD, Toshima-ku, Tokyo 171-8633 (JP); HORIE, Taro, c/o TAISHO PHARMACEUTICAL CO. LTD., Toshima-ku, Tokyo 171-8633 (JP)
(74) Representative: HOFFMANN EITLE
(86) International application number: PCT/JP2001/002951
(87) International publication number: WO 2001/076605

(56) References cited:
- EP-A- 0 312 412
- EP-A- 0 327 263
- EP-A- 0 376 821
- FR-A- 2 601 359
- FR-A- 2 756 561
- JP-A- 3 176 413
- JP-A- 9 188 620
- JP-A- 11 349 451

## Description

### TECHNICAL FIELD

The present invention relates to a minoxidil-containing composition which is prevented from coloring with time.

### BACKGROUND ART

Minoxidil is a component which is expected as an external hair growth agent because of its hair growth effect.

However, minoxidil, when stored as a solution, has a drawback of a tendency to color with time, and there is a potential risk that the deterioration of commodity value is caused thereby.

### PROBLEM TO BE SOLVED BY THE INVENTION

An object of the present invention is to prevent a minoxidil-containing composition from coloring.

### DISCLOSURE OF THE INVENTION

Generally, in order to incorporate a component having a tendency to color into a liquid preparation, it is possible to improve the anti-coloring property of the preparation by incorporating an anti-oxidant, such as dibutylhydroxytoluene thereinto. However, the present inventors have found that, in case of the minoxidil-containing liquid preparation, the combination of an anti-oxidant, i.e. one or two members selected from butylhydroxyanisole and dibutylhydroxytoluene therewith hardly contributes to prevention of coloring, or enhances coloring rather.

As a result of extensive researches in order to solve the problem, it has been found out that, when a minoxidil-containing liquid composition is adjusted to a pH of from 5.5 to 7.0, the above-mentioned disadvantage is improved and the prevention effect of coloring of the liquid preparation is remarkably elevated, and thereby the present invention has been accomplished.

That is, the present invention is directed to a minoxidil-containing composition which comprises an anti-oxidant and has a pH of from 5.5 to 7.0.

Further, the present invention is directed to a minoxidil-containing composition which comprises dibutylhydroxytoluene or butylhydroxyanisole and has a pH of from 5.5 to 7.0.

Furthermore, the present invention is directed to a method for preventing a minoxidil-containing composition from coloring, which comprises incorporating one or two members selected from dibutylhydroxytoluene and butylhydroxyanisole into the composition and adjusting the composition to a pH of from 5.5 to 7.0.

### MODE FOR CARRYING OUT THE INVENTION

The anti-oxidant to be used in the present invention is one or two members selected from the group consisting of dibutylhydroxytoluene, butylhydroxyanisole. Furthermore, a herbal extract can be also used as the anti-oxidant, and it is preferably one or two members selected from the group consisting of extracts of clove, geranium herb, rhubarb, phellodendron bark, peony root, moutan bark, coptis rhizome, citrus unshiu peel, hops, scutellaria root, cinnamon bark, glycyrrhiza, platycodon root, ginger, safflower and Japanese angelica root. These herbal extracts can be prepared by using water, an alcohol having 2 to 4 carbon atoms (e.g., ethanol, propylene glycol, 1,3-buthylene glycol or glycerol) or a mixture thereof as an extracting solvent.

The amount of the anti-oxidant in the present composition ranges preferably from 0.005 to 0.5 part by mass based on one part by mass of minoxidil, and especially preferably from 0.01 to 0.5 part by mass. Depending on the kinds of the anti-oxidant, addition of an insufficient amount of the anti-oxidant may sometimes enhance coloring, even if the pH falls within the range of from 5.5 to 7.0.

In the composition of the present invention, the prevention effect of coloring becomes low as the pH becomes low, so that the pH is preferably adjusted to 5.5 or higher, and especially preferably 5.7 or higher.

From the viewpoint of the solubility of minoxidil, the pH is preferably adjusted to 7.0 or lower, and especially preferably 6.3 or lower.

The pH regulation of the composition of the present invention can be carried out by using a conventional pH regulator such as, for example, an organic acid (e.g., citric acid, malic acid and lactic acid), or an inorganic acid (e.g., phosphoric acid, hydrochloric acid and sulfuric acid).

In the composition of the present invention, the concentration of minoxidil ranges from 3 to 6% by mass based on the whole composition.

The composition of the present invention can preferably contain a polyhydric alcohol as a solvent.

As the polyhydric alcohol, preferred are propylene glycol, dipropylene glycol, 1,3-butylene glycol, glycerol, hexylene glycol and polyethylene glycol, and more preferred is 1,3-butylene glycol. As the polyethylene glycol, preferred are Macrogol 200 and Macrogol 400. The amount incorporated of the polyhydric alcohol preferably ranges from 1 to 50 parts by mass based on one part by mass of minoxidil.

Preparation forms of the composition of the present invention, but not particularly limited thereof, can be any of lotions, aerosols and gels, and the production thereof can be carried out according to general methods for the preparations of medicines or cosmetics.

The composition of the present invention, if necessary, can contain solubilizers (e.g., fatty acid esters, polyhydric alcohol fatty acid esters, medium chain fatty acid glycerides, vegetable oils or alkyl glycerol ethers), surface active agents (e.g., nonionic surface active agents, lecithin derivatives or polymer emulsifiers), emulsifying stabilizers, absorption promoters, etc.

### ADVANTAGES OF THE INVENTION

The present invention makes it possible to provide a minoxidil-containing composition which can be prevented from coloring for a long period of time.

### EMBODIMENTS

The present invention will be illustrated in more detail by the following examples and experiments. The pH is determined for 5 mL of the preparation by means of a pH meter with a glass electrode (manufactured by DKK-Toa Corp.) in 5 minutes.

### Example 1

Phosphoric acid (0.5 mL) was added to minoxidil (5 g), 1,3-butylene glycol (30 g), ethanol (50 g) and dibutylhydroxytoluene (0.5 g), and the mixture was made up to the total volume of 100 ml with purified water, and dissolved with stirring to give a lotion-type composition of which the pH was 6.0.

### Example 2

Phosphoric acid (0.9 mL) was added to minoxidil (5 g), 1,3-butylene glycol (30 g), ethanol (50 g) and dibutylhydroxytoluene (0.1 g), and the mixture was made up to the total volume of 100 ml with purified water, and dissolved with stirring to give a lotion-type composition of which the pH was 5.5.

### Example 3

Citric acid (0.55 g) was added to minoxidil (5 g), 1,3-butylene glycol (30 g), ethanol (50 g) and dibutylhydroxytoluene (0.5 g), and the mixture was made up to the total volume of 100 ml with purified water, and dissolved with stirring to give a lotion-type composition of which the pH was 6.4.

### Example 4

Phosphoric acid (0.02 mL) was added to minoxidil (3 g), 1,3-butylene glycol (30 g), ethanol (50 g) and butylhydroxyanisole (0.5 g), and the mixture was made up to the total volume of 100 ml with purified water, and dissolved with stirring to give a lotion-type composition of which the pH was 7.0.

### Example 5

Phosphoric acid (0.3 mL) was added to minoxidil (5 g), propylene glycol (52 g), ethanol (25 g) and dibutylhydroxytoluene (0.5 g), and the mixture was made up to the total volume of 100 ml with purified water, and dissolved with stirring to give a lotion-type composition of which the pH was 6.0.

### Comparative Example 1

Purified water was added to minoxidil (3 g), 1,3-butylene glycol (30 g), ethanol (50 g) and dibutylhydroxytoluene (0.5 g) to be made up to the total volume of 100 ml, and the mixture was dissolved with stirring to give a lotion-type composition of which the pH was 8.8.

### Comparative Example 2

Minoxidil (5 g), propylene glycol (52 g) and ethanol (25 g) were mixed, and the mixture was made up to the total volume of 100 ml with purified water, and dissolved with stirring to give a lotion-type composition of which the pH was 8.7.

### Comparative Example 3

Minoxidil (5 g), propylene glycol (52 g), ethanol (25 g) and phosphoric acid (0.3 mL) were mixed, and the mixture was made up to the total volume of 100 ml with purified water, and dissolved with stirring to give a lotion-type composition of which the pH was 6.0.

### Comparative Example 4

Minoxidil (5 g), propylene glycol (52 g), ethanol (25 g) and dibutylhydroxytoluene (0.5 g) were mixed, and the mixture was made up to the total volume of 100 ml with purified water, and dissolved with stirring to give a lotion-type composition of which the pH was 8.7.

### Experiment 1

The compositions of Examples 1 to 4 and Comparative Example 1 were each poured into a PET bottle and stored at 50°C for one month, and the appearance (color tone) was observed. The appearance was evaluated according to the following standards.
0: No coloring is found, and there is no problem as goods.
1: Slight coloring is found, but there is no problem as goods.
2: Coloring is found, and there is a problem as goods.

The results are shown in Table 1.

**Table 1**

| Preparatios | Example 1 | Example 2 | Example 3 | Example 4 | Comparative Example 1 |
|---|---|---|---|---|---|
| PH | 6 | 5.5 | 6.4 | 7 | 8.8 |
| Evaluation | 0 | 1 | 0 | 1 | 2 |

Coloring is found in Comparative Example 1, and therefore it is judged that there is a problem to commercialization. In the compositions of Examples 1 to 4, no or slight coloring is found, and it is judged that there is no problem as goods.

### Experiment 2

The compositions of Example 5 and Comparative Examples 2 to 4 were each poured into a PET bottle and, after storing at 50°C for one month, the absorbance (420 nm) was measured by using an absorptiometer (manufactured by Beckman Co.).

The results are shown in Table 2.

**Table 2**

| Preparations | Example 5 | Comparative Example 2 | Comparative Example 3 | Comparative Example 4 |
|---|---|---|---|---|
| PH | 6 | 8.7 | 6 | 8.7 |
| BHT | 0.5 g | 0 g | 0 g | 0.5 g |
| Absorbance (420 nm) | 0.013 | 0.021 | 0.058 | 0.039 |

The results in Comparative Examples 2 and 3 show that coloring of the minoxidil-containing compositions is enhanced by lowering the pH. Furthermore, results in Comparative Example 4 show that coloring is enhanced by addition of BHT, not to mention it is prevented.

In contrast, there is obtained a superior prevention effect of coloring in Example 5 in comparison with any Comparative Examples.

## Claims

1. A minoxidil-containing composition which comprises one or two members selected from the group consisting of dibutylhydroxytoluene and butylhydroxyanisole, has a minoxidil concentration of from 3 to 6% by mass, and has a pH of from 5.5 to 7.0.

2. The minoxidil-containing composition according to claim 1, which has a pH of from 5.7 to 6.3.

3. The minoxidil-containing composition according to any one of claims 1 to 2, which comprises the one or two members selected from the group consisting of dibutylhydroxytoluene and butylhydroxyanisole in an amount of from 0.005 to 0.5 part by mass based on one part by mass of minoxidil.

4. The minoxidil-containing composition according to any one of claims 1 to 3, which further comprises a polyhydric alcohol.

5. The minoxidil-containing composition according to any one of claims 1 to 3, which further comprises polyethylene glycol.

6. A method for preventing a minoxidil-containing composition from coloring, which comprises incorporating one or two members selected from the group consisting of dibutylhydroxytoluene and butylhydroxyanisole in the composition that has a minoxidil concentration of from 3 to 6% by mass, and adjusting the composition to a pH of from 5.5 to 7.0.

## Patentansprüche

1. Minoxidilhaltige Zusammensetzung, die ein oder zwei Elemente, ausgewählt aus Dibutylhydroxytoluol und Butylhydroxyanisol, umfasst, eine Minoxidilkonzentration von 3-6 Gew.% aufweist und einen pH-Wert von 5,5-7,0 hat.

2. Minoxidilhaltige Zusammensetzung gemäss Anspruch 1, die einen pH-Wert von 5,7-6,3 hat.

3. Minoxidilhaltige Zusammensetzung gemäss mindestens einem der Ansprüche 1 bis 2, die ein oder zwei Elemente, ausgewählt aus Dibutylhydroxytoluol und Butylhydroxyanisol, in einer Menge von 0,005-,5 Gew.-Teilen, auf Basis von 1 Gew.-Teil Minoxidil, umfasst.

4. Minoxidilhaltige Zusammensetzung gemäss mindestens einem der Ansprüche 1 bis 3, die ferner einen mehrwertigen Alkohol umfasst.

5. Minoxidilhaltige Zusammensetzung gemäss mindestens einem der Ansprüche 1 bis 3, die ferner Polyethylenglykol umfasst.

6. Verfahren zur Verhinderung der Verfärbung einer minoxidilhaltigen Zusammensetzung, das die Inkorporierung von einem oder zwei Element(en), ausgewählt aus Dibutylhydroxytoluol und Butylhydroxyanisol, in eine Zusammensetzung, die eine Minoxidilkonzentration von 3-6 Gew.% aufweist, und die Einstellung der Zusammensetzung auf einen pH-Wert von 5,5-7,0 umfasst.

## Revendications

1. Composition contenant du minoxidil qui comprend un ou deux élément(s) choisi(s) parmi le groupe constitué du dibutylhydroxytoluène et. du butylhydroxyanisole, a une concentration en minoxidil de 3 à 6 % en masse, et a un pH de 5,5 à 7,0.

2. Composition contenant du minoxidil selon la revendication 1, qui a un pH de 5,7 à 6,3.

3. Composition contenant du minoxidil selon l'une quelconque des revendications 1 à 2, qui comprend l'un ou les deux élément(s) choisi(m) parmi le groupe constitué du dibutylhydroxytoluène et du butylhydroxyanisole en une quantité de 0,005 à 0,5 partie en masse basée sur une partie en masse de minoxidil.

4. Composition contenant du minoxidil selon l'une quelconque des revendications 1 à 3, qui comprend en outre un alcool polyhydrique.

5. Composition contenant du minoxidil selon l'une quelconque des revendications 1 à 3, qui comprend en outre du polyéthylène glycol.

6. Procédé pour empêcher une composition contenant du minoxidil de se colorer, qui comprend l'incorporation d'un ou deux élément(s) choisi(s) parmi le groupe constitué du dibutylhydroxytoluène et du butylhydroxyanisole dans la composition qui a une concentration en minoxidil de 3 à 6 % en masse, et l'ajustement de la composition à un pH de 5,5 à 7,0.
